# EUROPEAN PATENT APPLICATION

(11) **EP 3 804 747 A1**
(43) Date of publication of application: **14.04.2021**
(21) Application number: 20189367.4
(22) Date of filing: 15.01.2016
(51) Int. Cl.: A61K 38/18, A61K 47/12, A61P 25/04, A61K 9/00

(54) **THERAPEUTIC PROTEIN FORMULATIONS**

(30) Priority: 18.01.2015 US 201562104804 P
(62) Divisional of application: 16703658.1
(71) Applicant: Gloriana Therapeutics, Inc., Rhode Island, RI 02885 (US)
(72) Inventor: DIMITROVA, Mariana, Medford, MA Massachusetts 02155 (US)
(74) Representative: Grund, Martin

(57) **Abstract**

Systems and methods for preparing a therapeutic protein solution, reducing its viscosity, and administering it to a subject are provided herein. Methods provided herein are particularly useful for preparing a therapeutically effective concentration of Neublastin for subject administration.

## Description

### FIELD OF THE INVENTION

The present disclosure relates, in some aspects, to the field to therapeutic protein formulations.

### BACKGROUND OF INVENTION

Therapeutic proteins are under early evaluation for the treatment of neuropathic pain (e.g. sciatica, diabetic neuropathy, etc.). However, current formulations present several challenges to administration, particularly via subcutaneous injection.

### SUMMARY OF INVENTION

Aspects of the disclosure relate to compositions and methods that enable effective administration of therapeutic proteins for which high viscosity, poor solubility and/or phase separation limits their delivery by traditional approaches. In some embodiments, high viscosity of therapeutic protein formulations is a particular challenge when the mode of administration is subcutaneous injection, which often calls for therapeutically effective concentrations of protein to be present in a small volume (∼1 mL), at a viscosity that allows the formulations to flow easily through a relatively small orifice, such as that of a small gauge needle. Aspects of the disclosure relate to liquid formulations of Neublastin, and in particular liquid formulations that can be used for delivering small volumes of high concentration Neublastin (e.g., subcutaneously). Neublastin is a therapeutic protein used to treat neuropathic pain, which has a relatively low potency and poor subcutaneous bioavailability. Therefore, large doses of Neublastin are often called for to achieve desired therapeutic effects. However, conventional formulations containing succinate limit the solubility of Neublastin to 40 mg/mL under typical conditions, *e.g*., pH, temperature, *etc.* It has been found that at higher concentrations of Neublastin, the viscosity of the succinate formulations can become unacceptably high and therefore not compatible with certain modes of administration such as subcutaneous injection. Therefore, in some aspects, the present disclosure relates to compositions comprising Neublastin and methods of manufacturing Neublastin with significantly improved solubility and decreased viscosity. In particular it has been found that formulations of Neublastin containing citrate offer improved solubility and viscosity compared to the currently used succinate formulations.

Accordingly, in one aspect, the disclosure relates to a pharmaceutical formulation comprising Neublastin and citrate. In some embodiments, a formulation has a viscosity suitable for subcutaneous injection. In some embodiments, a formulation has a viscosity suitable for injection through a needle in a range of 29 gauge to 31 gauge in size. In some embodiments, a formulation has a viscosity suitable for injection through a needle in a range of 29 gauge to 31 gauge in size while at a temperature in range of 40-60 °C. In some embodiments, a formulation has a viscosity of less than 35 cP at Neublastin concentrations of up to 150 mg/mL. In some embodiments, a formulation has a viscosity of up to about 20 cP at Neublastin concentrations of up to 135 mg/mL.

In some embodiments, Neublastin is at a concentration in a formulation of up to 150 mg/mL. In some embodiments, Neublastin is at a concentration in a formulation in a range of above 40 mg/mL to 150 mg/mL. In some embodiments, citrate is at a concentration of 50 mM to 150 mM. In some embodiments, citrate comprises citrate at a concentration of 75 mM to 100 mM

In some aspects, the disclosure relates to a method of manufacturing Neublastin. In some embodiments, methods provided herein involve combining Neublastin with citrate.

In some embodiments, the step of combining Neublastin with the citrate results in a Neublastin concentration of up to 150 mg/mL in the combination. In some embodiments, the step of combining Neublastin citrate results in a Neublastin concentration of up to 135 mg/mL in the combination. In some embodiments, the step of combining Neublastin with citrate results in a Neublastin concentration of above 40 mg/mL to 150 mg/mL in the combination.

In some embodiments, the step of combining Neublastin with citrate results in a citrate concentration of 50 mM to 150 mM in the combination. In some embodiments, the step of combining Neublastin with citrate results in a citrate concentration of 75 mM to 100 mM in the combination.

In some embodiments, the step of combining Neublastin with citrate results in the combination having a viscosity of up to 100 cP, for example 50-100 cP, around 50 cP, less than 50 cP, 40-50 cP, around 40 cP, less than 40 cP, 20-40 cP, or around 35 cP. In some embodiments, the step of combining Neublastin with citrate results in the combination having a viscosity of up to 20 cP. In some embodiments, the step of combining Neublastin with citrate results in the combination having a viscosity of up to 35 cP at room temperature.

In some embodiments, the method further comprises filtering the combination of Neublastin and citrate at a temperature in a range of 30°C to 50°C. In some embodiments, the step of filtering comprises performing ultrafiltration. In some embodiments, the step of filtering comprising performing diafiltration.

The present disclosure also provides a method of administering Neublastin to treat neuropathic pain by injecting higher efficacious doses into the SC interstitial space due to the improved physicochemical properties and flow dynamics of the product (reduced viscosity and improved solubility). Accordingly, in some aspects, the disclosure relates to a method of treating neuropathic pain in a subject, the method comprising subcutaneously administering a pharmaceutical formulation comprising a therapeutically effective amount of Neublastin and citrate to the subject.

In some embodiments of the method, the pharmaceutical formulation is administered through a needle of 29 gauge to 31 gauge in size. In some embodiments of the method, the formulation is administered through a needle of 29 gauge to 31 gauge in size at a temperature in range of 40°C to 60°C.

In some embodiments of the method, the formulation has a viscosity of less than 35 cP at Neublastin concentrations up to 150 mg/mL. In some embodiments of the method, the formulation has a viscosity of up to about 20 cP at Neublastin concentrations up to 135 mg/mL.

In some embodiments of the method, the Neublastin is at a concentration in the formulation of up to 150 mg/mL. In some embodiments of the method, the Neublastin is at a concentration in the formulation in a range of above 40 mg/mL to 150 mg/mL. In some embodiments, the formulation comprises citrate at a concentration of 50 mM to 150 mM. In some embodiments of the method, the formulation comprise citrate at a concentration of 75 mM to 100 mM

Administration of the pharmaceutical formulations described herein can be further enhanced by heating the formulation over a range of temperatures, and preferably about 50°C without causing a negative impact on Neublastin's integrity due to its unique thermodynamic properties (very high melting temperatures). Therefore, in some aspects, the disclosure relates to a method of administering Neublastin to a subject, the method comprising subcutaneously administering a pharmaceutical formulation comprising Neublastin and citrate; and controlling the temperature of the formulation such that the formulation is at a temperature in a range of 30 °C to 60 °C during the subcutaneous administration. In some embodiments of the method, the temperature is controlled such that it is in a range of 40 °C to 50 °C during the subcutaneous administration.

In some embodiments of the method, the formulation is subcutaneously administered by injecting the formulation through a needle. In some embodiments of the method, the formulation is subcutaneously administered by injecting the formulation through a needle of a syringe by manually displacing a plunger of the syringe. In some embodiments of the method, the formulation is subcutaneously administered by injecting the formulation through a needle that is fluidically connected to a pump configured for dispensing the formulation.

In some embodiments of the method, the formulation is self-administered by the subject. In some embodiments of the method, the formulation is administered by a health care provider. In some embodiments of the method, the formulation is administered for purposes of treating neuropathic pain.

In some aspects, the disclosure relates to a pharmaceutical formulation comprising Neublastin and a citrate salt in a lyophilized form.

In some aspects, the disclosure relates to a method comprising determining the extent to which the relative aqueous insolubility of a polypeptide at particular pH is associated with dipole-dipole interactions of the polypeptide, wherein the particular pH is below the isoelectric point of the polypeptide or above the isoelectric point of the polypeptide; and if the relative insolubility is significantly associated with dipole-dipole interactions, combining the polypeptide with citrate to enhance the solubility of the polypeptide.

In some embodiments of the method, the dipole-dipole interactions are intramolecular interactions. In some embodiments of the method, the dipole-dipole interactions are intermolecular interactions.

### BRIEF DESCRIPTION OF DRAWINGS

FIG. 1 depicts the charts demonstrating several characteristics of the Neublastin succinate formulation.
FIG. 2 shows the effects of pH (left) and protein concentration (right) on the Neublastin succinate formulation. The photo (far right) demonstrates that phase separation of Neublastin succinate formulation occurs due to insolubility.
FIG. 3 demonstrates that the viscosity of the Neublastin succinate formulation increases exponentially at protein concentrations > 50mg/mL. At 100 mg/mL, the viscosity is -25 cP (as a comparison, platform mAbs show viscosity < 10 cP at this concentration). Immediate phase separation occurs at 200 mg/mL. Phase separation occurs on a longer timescale at concentrations > 150 mg/mL.

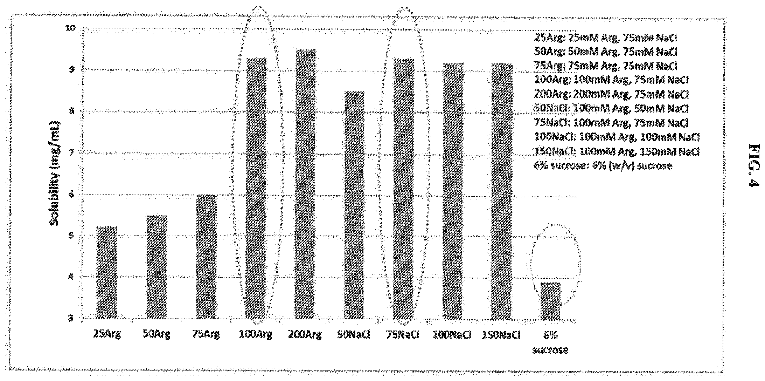

FIG. 4 depicts a chart demonstrating the effects of excipients on Neublastin solubility.
FIG. 5 shows data demonstrating that poor NBN solubility correlates with unfavorable colloidal stability (kD).
FIG. 6 shows tables depicting the design of experiment (DOE) parameters for the reformulation of Neublastin and a list of Neublastin formulations designed using the DOE parameters.
FIG. 7 is a graph demonstrating is a graph demonstrating that replacing the succinate buffer with citrate improves viscosity of Neublastin formulations.
FIG. 8 is a chart showing the different viscosities of various proteins at different concentrations. The viscosity of the succinate Neublastin formulation is greater at the same protein concentration as the citrate Neublastin formulation.
FIG. 9 depicts charts showing the improved solubility, colloidal and conformational stability of Neublastin in citrate compared to Neublastin in succinate buffer.
FIG. 10 depicts the data demonstrating that replacement of succinate buffer with citrate results in 8X increased solubility of Neublastin.

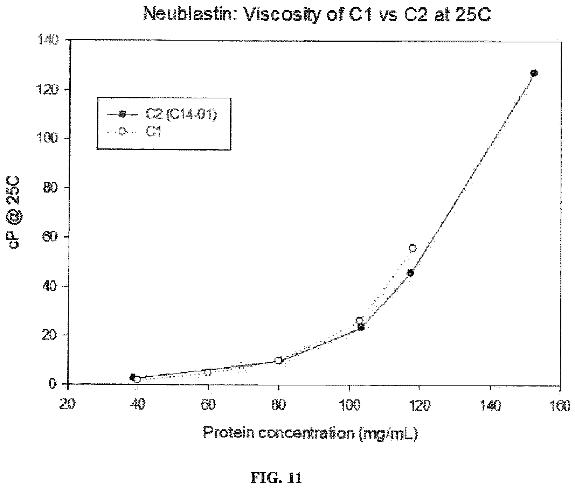

FIG. 11 shows the Neublastin in succinate buffer formulation has comparable viscosities before (C1) and after (C2) a freeze-thaw cycle.

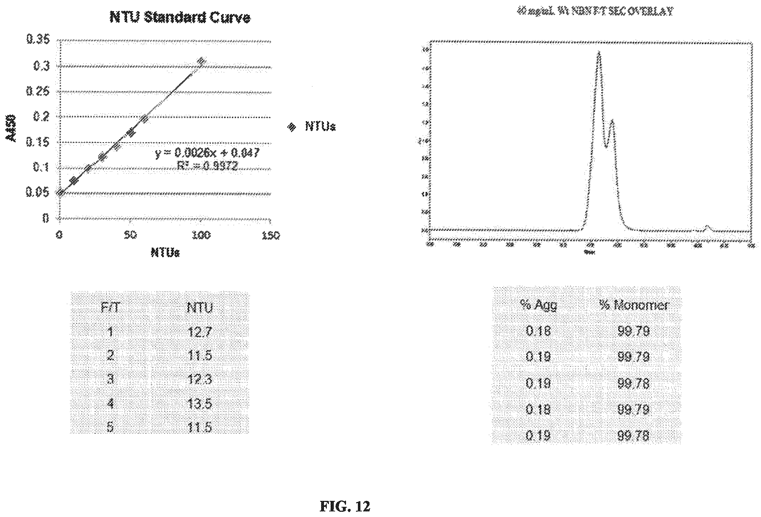

FIG. 12 depicts data demonstrating 5X Freeze Thaw data at 40 mg/mL.
FIG. 13 shows viscosity trends for both the Neublastin in succinate buffer and Neublastin and citrate formulations at a range of temperatures and protein concentrations.
FIG. 14 demonstrates that improved Neublastin solubility correlates with significantly minimized viscosity.

### DETAILED DESCRIPTION OF DISCLOSURE

Neublastin (NBN), also known as artemin, is a protein encoded in humans by the *ARTN* gene. Neublastin is a neurotrophin, or neuronal growth factor, and is classified as a glial-derived neurotrophic factor (GDNF) ligand family. In some embodiments, Neublastin acts specifically on sensory neurons, promoting their survival and regrowth. Therefore, in some aspects, Neublastin may be useful for the treatment of neuropathic pain.

Aspects of the disclosure relate to liquid formulations of Neublastin that can support relatively high concentrations of Neublastin in a form that is acceptable for low volume delivery to a subject (e.g., via subcutaneous administration). Neublastin has a relatively low potency and poor subcutaneous bioavailability, and consequently, a large dose (>100 mg) is required to achieve a therapeutic effect. An additional challenge with Neublastin is the very high viscosity of the protein compared to other molecules. As Neublastin is intended to treat chronic pain, a treatment modality based on regular patient self-administration via subcutaneous injection is highly desirable. For subcutaneous dosing to be considered viable for self-administration, the injection volume should be no more than about 2 mL (e.g., 1-2 mL, about 1 mL or less than 1mL), and the viscosity must be low enough for the solution to flow easily through a fine gauge needle without requiring an excessively high force. In some cases, high viscosity is the single limiting factor preventing a therapeutically effective molecule from being a therapeutically viable product.

The viscosity of Neublastin becomes unacceptably high at concentrations greater than about 40 mg/mL in succinate and other formulations tested. Consequently, current formulation technology limits NBN to a -40 mg/mL concentration in the final drug product which significantly limits the amount that can be administered as a bolus injection in the subcutaneous (SC) space.

### Pharmaceutical formulations and methods of manufacture

In some aspects, the present disclosure relates to the surprising discovery that formulations of Neublastin comprising citrate, *e*.*g*., as a buffering agent, offer improved solubility and viscosity compared to the currently used succinate-based formulations. As used herein, the term "citrate" refers to a derivative of citric acid found in solution. In some embodiments, citrate is a salt, ester, and/or a polyatomic anion. In some embodiments, citrate is provided with a sodium adduct, such as in the form of trisodium citrate. In some embodiments, citrate is provided in solution at an appropriate pH (*e.g*., a pH in a range of 3.0 to 6.2) by combining citric acid monohydrate with trisodium citrate dihydrate in solution. In some embodiments, citrate is provided in solution at an appropriate pH (*e.g.,* a pH in a range of 2.6 to 7.6) by combining citric acid and Na₂HPO₄ in solution. Other suitable sources of citrate may be used, including any suitable monobasic, dibasic, or tribasic form. In some embodiments of formulations described herein, citrate is present at a concentration ranging from 1 mM to 200 mM, 10 mM to 175 mM, 50 mM to 150 mM, or 75 mM to 100 mM. In some embodiments, citrate is present at a concentration of about 75 mM, 80 mM, 85 mM, 90 mM, 95 mM or 100 mM

Accordingly, in some embodiments, pharmaceutical formulations or compositions disclosed herein include a buffer (*e*.*g*., a citrate buffer). As used herein, the term "buffer" refers to a pH-controlled medium. In some embodiments, a buffer can be used to dissolve an active pharmaceutical ingredient (*e.g*., a therapeutic protein, such as Neublastin) and/or pharmaceutically acceptable salts and excipients, or as a diluent to dissolve liquid pharmaceutical compounds and compositions. Buffers neutralize amounts of acid or base present within a solution and, in doing so, maintain a stable pH of the solution within particular range. In some embodiments, the buffering capacity of a buffer is in a range of pH 3.0 to pH 8.0, pH 4.0 to pH 6.0, or pH 5.0 to pH 7.0. In some embodiments In some embodiments, the buffering capacity of a buffer is about pH 4.0, about pH 4.5, about pH 5.0, about pH 5.5, about pH 6.0 or about pH 6.5.

In some embodiments, the disclosure relates to a protein therapeutic (*e.g*. Neublastin) that is dissolved in a buffer having a pH that is above or below the isoelectric point (pI) of the protein therapeutic. As used herein, the term "isoelectric point" refers to the pH at which a particular molecule carries no net electrical surface charge. At a pH below their pI, proteins carry a net positive surface charge. At a pH above their pI, proteins carry a net negative surface charge. The surface charge of a protein affects its solubility by increasing or decreasing the amount of interaction that protein has with surrounding molecules (*e.g*. solvent molecules such as water and/or other protein molecules in solution). In some aspects the disclosure relates to the discovery that the charged nature of certain proteins, for example Neublastin, in the context of their pI, make them more viscous and less soluble in pharmaceutical formulations. In some embodiments, the instant disclosure provides a solution to these issues in the form of a pharmaceutical composition comprising Neublastin and citrate.

In some aspects, the present disclosure provides pharmaceutical formulations comprising Neublastin and citrate that have improved viscosity compared to other formulations (*e.g*. succinate) of Neublastin. As used herein, the term "viscosity" refers to a measure of fluids resistance to deformation, *e.g*., by a shear force or tensile force. Viscosity is often measured by a viscometer and expressed in centipoise (cP) units. In some embodiments, fluids having relative high viscosity display higher resistance to shear forces (*e.g*., are "thicker") and thus under a particular force will flow more slowly than fluid having relatively low viscosity. It should be appreciated that the viscosity of a liquid formulation can be measured using any suitable technique including using a rheometer, a viscometer (e.g., a capillary viscometer or microcapillary viscometer), or any other suitable device.

In some embodiments, order for a pharmaceutical formulation to be useful for subcutaneous injection, its viscosity must be low enough for the solution to flow easily through a needle or other similar conduit or orifice without requiring an excessively high force. In some embodiments, pharmaceutical formulations comprising Neublastin and citrate, as described herein, have a viscosity suitable for subcutaneous injection. In some embodiments, the pharmaceutical formulation's viscosity is less than 100 centipoise (cP). In some embodiments, the pharmaceutical formulation's viscosity is in a range of 1 cP and 100 cP. In some embodiments, the pharmaceutical formulation's viscosity is in a range of 10 cP to 50 cP. In some embodiments, the pharmaceutical formulation's viscosity is in a range of 15 cP to 35 cP. In some embodiments, the pharmaceutical formulation's viscosity is 15 cP, 16 cP, 17 cP, 18 cP, 19 cP, 20 cP, 21 cP, 22 cP, 23 cP, 24 cP, 25 cP, 26 cP, 27 cP, 28 cP, 29 cP, 30 cP, 31 cP, 32 cP, 33 cP, 34 cP, 35 cP, 36 cP, 37 cP, 38 cP, 39 cP, or 40 cP. In some embodiments, a formulation has a viscosity suitable for injection through a needle or other suitable device (*e.g*. a catheter) in a range of 27 gauge to 31 gauge (e.g., 29 gauge to 31 gauge) in size. In some embodiments, the formulation has a viscosity suitable for injection through a needle or other suitable device having an inner diameter of 0.22 mm, 0.20 mm, 0.18 mm, 0.16 mm, 0.14 mm, 0.12 mm, or 0.10 mm. In some embodiments, the formulation has a viscosity suitable for injection through a needle or other suitable device having an inner diameter in a range of 0.05 mm to 0.50 mm, 0.10 mm to 0.30 mm or 0.10 mm to 0.20 mm.

In some embodiments, the formulation has a viscosity suitable for injection through a needle or other suitable device in a range of 27 gauge to 31 gauge (e.g., 29 gauge to 31 gauge) in size while at room temperature (for example from 15-30 °C, e.g., between 20 °C and 25 °C) or when administered at higher than room temperature, for example in a temperature in range of 40-60 °C. In some embodiments, the formulation has a viscosity suitable for injection through a needle or other suitable device having a gauge selected from the group consisting of 27 gauge, 28 gauge, 29 gauge, 30 gauge and 31 gauge, while at room temperature or at a temperature in a range of 40-60 °C. In some embodiments, the formulation has a viscosity suitable for injection through a needle or other suitable device having an inner diameter selected from the group consisting of 0.18 mm, 0.16 mm, and 0.14 mm, while at room temperature or at a temperature in a range of 40-60 °C. In some embodiments, a formulation has a viscosity of less than 35 cP at Neublastin concentrations of up to 150 mg/mL. In some embodiments, the formulation has a viscosity of up to about 20 cP at Neublastin concentrations of up to 135 mg/mL.

It should be appreciated that in some embodiments compositions and formulations described herein can be administered by any suitable route, including but not limited to one of: intravenous injection or infusion (IV), subcutaneous injection (SC), intraperitoneally (IP), or intramuscular injection. It is also possible to use intra-articular delivery. Other modes of parenteral administration can also be used. Examples of such modes include: intraarterial, intrathecal, intracapsular, intraorbital, intracardiac, intradermal, transtracheal, subcuticular, intraarticular, subcapsular, subarachnoid, intraspinal, and epidural and intrasternal injection.

Other aspects of the disclosure relate to the increased concentration of Neublastin that is able to be solubilized in a formulation comprising citrate. The increased solubility of Neublastin in the formulations described herein allows for therapeutically effective amounts of Neublastin to be delivered in a reduced volume compared to conventional (*e.g*., succinate) formulations.

As used herein, the term "therapeutically effective" refers to the amount of an active pharmaceutical ingredient (*e.g*., Neublastin) required to prevent, treat or reduce symptoms associated with a particular disease or disorder (for example, neuropathic pain). A therapeutically effective amount of a therapeutic protein, such as Neublastin, may vary according to factors such as the disease state, age, sex, and weight of the individual, and the ability of the protein to elicit a desired response in the individual, *e.g*., amelioration of at least one disorder parameter or amelioration of at least one symptom of the disorder. A therapeutically effective amount is also one in which any toxic or detrimental effects of the protein are outweighed by the therapeutically beneficial effects.

Thus, in some embodiments of formulations described herein, Neublastin is present at a concentration in a range of 10 mg/mL to 300 mg/mL. In some embodiments, Neublastin is present at a concentration in a range of 30 mg/mL to 200 mg/mL. In some embodiments, Neublastin is present at a concentration in a range of 40 mg/mL to 175 mg/mL. In some embodiments, Neublastin is present at a concentration of 50 mg/mL. In some embodiments, Neublastin is present at a concentration of 60 mg/mL. In some embodiments, Neublastin is present at a concentration of 70 mg/mL. In some embodiments, Neublastin is present at a concentration of 80 mg/mL. In some embodiments, Neublastin is present at a concentration of 90 mg/mL. In some embodiments, Neublastin is present at a concentration of 100 mg/mL. In some embodiments, Neublastin is present at a concentration of 110 mg/mL. In some embodiments, Neublastin is present at a concentration of 120 mg/mL. In some embodiments, Neublastin is present at a concentration of 130 mg/mL. In some embodiments, Neublastin is present at a concentration of 140 mg/mL. In some embodiments, Neublastin is present at a concentration of 150 mg/mL.

In some embodiments, Neublastin compositions or formulations described herein are administered to a subject (e.g., subcutaneously). A subject can be a vertebrate, mammal, including but not limited to, a human, a non-human primate, a rodent, an ovine, a bovine, or other mammal. A subject can be a subject in need of treatment with Neublastin. In some embodiments a small volume of a Neublastin liquid formulation (e.g., 0.5-2.0 mL, or around 1mL) containing a relatively high concentration of Neublastin (e.g., over 50 mg/mL, around 100 mg/mL or more, 100-150 mg/mL, around 150 mg/mL, 150-200 mg/mL, around 200 mg/mL, 200-250 mg/mL, around 250 mg/mL, 250-350 mg/mL, 350-450 mg/mL, or more) having a viscosity of less than 100 cP (e.g., less than 50 cP) is administered to a subject. It should be appreciated that in some embodiments, formulations described herein can be used for small volume administration (e.g., between 0.5 mL and 2 mL, for example around 1 mL injection volumes). It also should be appreciated that in some embodiments, one or more formulations described herein can be sterilized using any suitable technique (e.g., filtration or other technique) in order to produce a composition that is suitable (e.g., sterile) for administration to a subject.

In some embodiments, the disclosure also provides methods for manufacturing pharmaceutical formulations. In some embodiments, the step of combining Neublastin with citrate results in a Neublastin concentration of up to 150 mg/mL in the combination. In some embodiments, the step of combining Neublastin with citrate results in a Neublastin concentration of up to 135 mg/mL in the combination. In some embodiments, the step of combining Neublastin with citrate results in a Neublastin concentration of above 40 mg/mL to 150 mg/mL in the combination.

In some embodiments, compositions comprising Neublastin and citrate comprise molecular aggregates and other unwanted components. It is therefore desirable in some embodiments to filter the combination to remove the unwanted components. In some embodiments, the step of filtering comprises performing ultrafiltration. In some embodiments, the step of filtering comprising performing diafiltration. Other methods of filtration are also contemplated herein, for example column chromatography (*e.g*. ion-exchange chromatography, affinity chromatography), microfiltration, membrane filtration, high performance tangential flow filtration (HPTFF), gel filtration, and nanofiltration.

In some embodiments, a composition comprising Neublastin and citrate is filtered at a temperature in a range of 10°C to 80°C, 20°C to 60°C or 30°C to 50°C. In some embodiments, a composition comprising Neublastin and citrate is filtered at a temperature of 25°C, 26°C, 27°C, 28°C, 29°C, 30°C, 31°C, 32°C, 33°C, 34°C, 35°C, 36°C, 37°C, 38°C, 39°C, 40°C, 41°C, 42°C, 43°C, 44°C, 45°C, 46°C, 47°C, 48°C, 49°C, or 50°C .

The present disclosure also contemplates pharmaceutical formulations comprising Neublastin and citrate and one or more pharmaceutically acceptable excipients. Pharmaceutically acceptable excipients include any and all solvents, diluents, or other liquid vehicles, dispersions, suspension aids, surface active agents, isotonic agents, thickening or emulsifying agents, preservatives, solid binders, lubricants, and the like, as suited to the particular dosage form desired. General considerations in formulation and/or manufacture of pharmaceutical compositions agents can be found, for example, in Remington's Pharmaceutical Sciences, Sixteenth Edition, E. W. Martin (Mack Publishing Co., Easton, Pa., 1980), and Remington: The Science and Practice of Pharmacy, 21st Edition (Lippincott Williams & Wilkins, 2005).

In some embodiments, the excipient is an amino acid. In some embodiments, the amino acid is arginine and/or lysine. In some embodiments, arginine and/or lysine is present at a concentration between 1 mM and 200 mM. In some embodiments, arginine and/or lysine is present at a concentration between 25 mM and 150 mM. In some embodiments, arginine and/or lysine is present at a concentration between 50 mM and 75 mM. In some embodiments, arginine and/or lysine is present at about 25 mM, about 30 mM, about 35 mM, about 40 mM, about 45 mM, about 50 mM, about 55 mM, about 60 mM, about 65 mM, about 70 mM, about 75 mM, about 80 mM, about 85 mM, about 90 mM, about 95 mM, about 100 mM, about 105 mM, about 110 mM, about 115 mM, about 120 mM, about 125 mM, about 130 mM, about 135 mM, about 140 mM, about 145 mM, about 150 mM, about 155 mM, about 160 mM, about 165 mM, about 170 mM, about 175 mM, about 180 mM, about 185 mM, about 190 mM, about 195 mM, or about 200 mM

Pharmaceutical formulations described herein can be prepared by any method known in the art of pharmacology. In general, such preparatory methods include the steps of bringing a compound described herein (the "active ingredient") into association with a carrier and/or one or more other accessory ingredients, and then, if necessary and/or desirable, shaping and/or packaging the product into a desired single- or multi-dose unit.

Pharmaceutical formulations can be prepared, packaged, and/or sold in bulk, as a single unit dose, and/or as a plurality of single unit doses. As used herein, a "unit dose" is discrete amount of the pharmaceutical composition comprising a predetermined amount of the active ingredient. The amount of the active ingredient is generally equal to the dosage of the active ingredient which would be administered to a subject and/or a convenient fraction of such a dosage such as, for example, one-half or one-third of such a dosage.

Relative amounts of the active ingredient, the pharmaceutically acceptable excipient, and/or any additional ingredients in a pharmaceutical composition of the present disclosure will vary, depending upon the identity, size, and/or condition of the subject treated and further depending upon the route by which the composition is to be administered. By way of example, the composition may comprise between 0.1% and 100% (w/w) active ingredient.

Pharmaceutically acceptable excipients used in the manufacture of provided pharmaceutical compositions include inert diluents, dispersing and/or granulating agents, surface active agents and/or emulsifiers, disintegrating agents, binding agents, preservatives, buffering agents, lubricating agents, and/or oils.

Examples of diluents include calcium carbonate, sodium carbonate, calcium phosphate, dicalcium phosphate, calcium sulfate, calcium hydrogen phosphate, sodium phosphate lactose, sucrose, cellulose, microcrystalline cellulose, kaolin, mannitol, sorbitol, inositol, sodium chloride, dry starch, cornstarch, powdered sugar, and mixtures thereof.

Examples of surface active agents and/or emulsifiers include natural emulsifiers (*e.g.*, acacia, agar, alginic acid, sodium alginate, tragacanth, chondrux, cholesterol, xanthan, pectin, gelatin, egg yolk, casein, wool fat, cholesterol, wax, and lecithin), colloidal clays (*e*.*g*., bentonite (aluminum silicate) and Veegum (magnesium aluminum silicate)), long chain amino acid derivatives, high molecular weight alcohols (*e.g.*, stearyl alcohol, cetyl alcohol, oleyl alcohol, triacetin monostearate, ethylene glycol distearate, glyceryl monostearate, and propylene glycol monostearate, polyvinyl alcohol), carbomers (*e.g*., carboxy polymethylene, polyacrylic acid, acrylic acid polymer, and carboxy vinyl polymer), carrageenan, cellulosic derivatives (*e.g.*, carboxymethylcellulose sodium, powdered cellulose, hydroxymethyl cellulose, hydroxypropyl cellulose, hydroxypropyl methylcellulose, methylcellulose), sorbitan fatty acid esters (*e.g.,* polyoxyethylene sorbitan monolaurate (Tween 20), polyoxyethylene sorbitan (Tween 60), polyoxyethylene sorbitan monooleate (Tween 80), sorbitan monopalmitate (Span 40), sorbitan monostearate (Span 60), sorbitan tristearate (Span 65), glyceryl monooleate, sorbitan monooleate (Span 80)), polyoxyethylene esters (*e*.*g*., polyoxyethylene monostearate (Myrj 45), polyoxyethylene hydrogenated castor oil, polyethoxylated castor oil, polyoxymethylene stearate, and Solutol), sucrose fatty acid esters, polyethylene glycol fatty acid esters (*e*.*g*., Cremophor™), polyoxyethylene ethers, (*e*.*g*., polyoxyethylene lauryl ether (Brij 30)), poly(vinyl-pyrrolidone), diethylene glycol monolaurate, triethanolamine oleate, sodium oleate, potassium oleate, ethyl oleate, oleic acid, ethyl laurate, sodium lauryl sulfate, Pluronic F68, Poloxamer 188, cetrimonium bromide, cetylpyridinium chloride, benzalkonium chloride, docusate sodium, and/or mixtures thereof.

Examples of preservatives include antioxidants, chelating agents, antimicrobial preservatives, antifungal preservatives, alcohol preservatives, acidic preservatives, and other preservatives.

Exemplary antioxidants include alpha tocopherol, ascorbic acid, acorbyl palmitate, butylated hydroxyanisole, butylated hydroxytoluene, monothioglycerol, potassium metabisulfite, propionic acid, propyl gallate, sodium ascorbate, sodium bisulfite, sodium metabisulfite, and sodium sulfite.

Examples of chelating agents include ethylenediaminetetraacetic acid (EDTA) and salts and hydrates thereof (*e.g*., sodium edetate, disodium edetate, trisodium edetate, calcium disodium edetate, dipotassium edetate, and the like), fumaric acid and salts and hydrates thereof, malic acid and salts and hydrates thereof, phosphoric acid and salts and hydrates thereof, and tartaric acid and salts and hydrates thereof. Exemplary antimicrobial preservatives include benzalkonium chloride, benzethonium chloride, benzyl alcohol, bronopol, cetrimide, cetylpyridinium chloride, chlorhexidine, chlorobutanol, chlorocresol, chloroxylenol, cresol, ethyl alcohol, glycerin, hexetidine, imidurea, phenol, phenoxyethanol, phenylethyl alcohol, phenylmercuric nitrate, propylene glycol, and thimerosal.

Examples of antifungal preservatives include butyl paraben, methyl paraben, ethyl paraben, propyl paraben, benzoic acid, hydroxybenzoic acid, potassium benzoate, potassium sorbate, sodium benzoate, sodium propionate, and sorbic acid.

Examples of alcohol preservatives include ethanol, polyethylene glycol, phenol, phenolic compounds, bisphenol, chlorobutanol, hydroxybenzoate, and phenylethyl alcohol.

Examples of acidic preservatives include vitamin A, vitamin C, vitamin E, beta-carotene, acetic acid, dehydroacetic acid, ascorbic acid, sorbic acid, and phytic acid.

Examples of other preservatives include tocopherol, tocopherol acetate, deteroxime mesylate, cetrimide, butylated hydroxyanisol (BHA), butylated hydroxytoluened (BHT), ethylenediamine, sodium lauryl sulfate (SLS), sodium lauryl ether sulfate (SLES), sodium bisulfite, sodium metabisulfite, potassium sulfite, potassium metabisulfite, Glydant Plus, Phenonip, methylparaben, Germall 115, Germaben II, Neolone, Kathon, and Euxyl. In certain embodiments, the preservative is an anti-oxidant. In other embodiments, the preservative is a chelating agent.

### Treatment of Neuropathic pain

Neuropathic pain is a complex, chronic pain state that is often characterized by tissue or nerve injury or dysfunction. Neuropathic pain is split into three classifications, peripheral neuropathic pain, central neuropathic pain and mixed (central and peripheral) neuropathic pain, depending upon the localization of the nerves affected. Neuropathic pain is associate with a myriad of causative agents, including but not limited to physical trauma (*e*.*g* spinal cord or nerve crush injuries), metabolic conditions (*e.g.* diabetes), viral infections (*e.g.* herpes or HIV infection), autoimmune disease (*e.g*. multiple sclerosis), cancer, toxins and nutritional deficiencies.

Aspects of the disclosure relate to the use of Neublastin for treatment of neuropathic pain. However, as discussed herein, the properties of conventional Neublastin formulations (e.g. succinate based forumlations), for example low solubility and high viscosity at therapeutically effective concentrations, present challenges for its clinical use in the treatment of neuropathic pain. The present disclosure, therefore, provides a method of treating neuropathic pain in a subject, the method comprising administering, *e.g*., subcutaneously, a pharmaceutical formulation comprising a therapeutically effective amount of Neublastin and citrate to the subject.

The formulations described herein have increased concentrations of Neublastin (due to the increased solubility of Neublastin in citrate) and decreased viscosity, thereby making them useful for subcutaneous administration (*i.e.* subcutaneous injection). Methods of subcutaneous injection are known in the art, for example as disclosed in The Textbook of Basic Nursing, 9th ed., Bunker Rosendahl and Kowalski (eds), Wolters Kluwer, Philadelphia, 2008. It is to be appreciated that any size needle (as measured by gauge or inner diameter) deemed clinically acceptable for use in subcutaneous injection can be used to administer a pharmaceutical formulation by the methods described herein. Examples of acceptable needle gauges include but are not limited to 20 gauge, 21 gauge, 22 gauge, 23 gauge, 24 gauge, 25 gauge, 26 gauge, 27 gauge, 28 gauge, 29 gauge, 30 gauge, and 31 gauge. In some embodiments of the method, the pharmaceutical formulation is administered through a needle of 29 gauge to 31 gauge in size. In some embodiments, the formulation has a viscosity suitable for injection through a needle or other suitable device (*e.g*. a catheter) having an inner diameter of 0.6 mm to 0.08 mm. In some embodiments, the formulation has a viscosity suitable for injection through a needle or other suitable device (*e.g*. a catheter) having an inner diameter of 0.34 mm to 0.16 mm. In some embodiments, the formulation has a viscosity suitable for injection through a needle or other suitable device (*e.g*. a catheter) having an inner diameter of 0.18 mm to 0.12 mm. In some embodiments, the formulation has a viscosity suitable for injection through a needle or other suitable device (*e.g*. a catheter) having an inner diameter selected from the group consisting of 0.67 mm. 0.60 mm, 0.52 mm, 0.51 mm, 0.41 mm, 0.34 mm, 0.31 mm, 0.26 mm, 0.21 mm, 0.18 mm, 0.16 mm, 0.12 mm, 0.11 mm, and 0.8 mm.

### EXAMPLES

### Example: Improving solubility of Neublastin with citrate leads to significant decrease of viscosity

### Challenges presented by succinate formulation of Neublastin

Two main challenges in the administration of therapeutically effective amounts of Neublastin are its low solubility and high viscosity at bioactive concentration levels. This example describes how the present disclosure overcomes the challenges of delivering therapeutically relevant doses of Neublastin via subcutaneous injection.

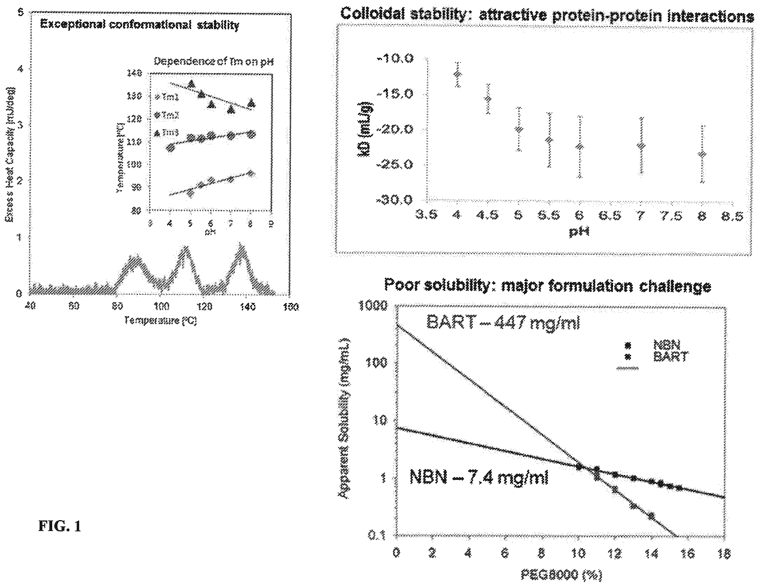

The formulation of Neublastin currently in clinical use comprises Neublastin, sodium succinate, L-Arginine/HCl and NaCl at a pH of 5.5 (referred to herein as the "succinate formulation"). As shown in FIG. 1, the succinate formulation displays exceptional thermal stability across a range of temperatures. However, the succinate formulation of Neublastin also has unfavorable colloidal stability (as measured by diffusion interaction parameter; k_{D}) and poor solubility (FIG. 1), making the delivery of pharmaceutically effective doses a challenge. This is because Neublastin has low potency and poor bioavailability in the subcutaneous space, meaning that a large dose of Neublastin is required to exert a therapeutic effect.

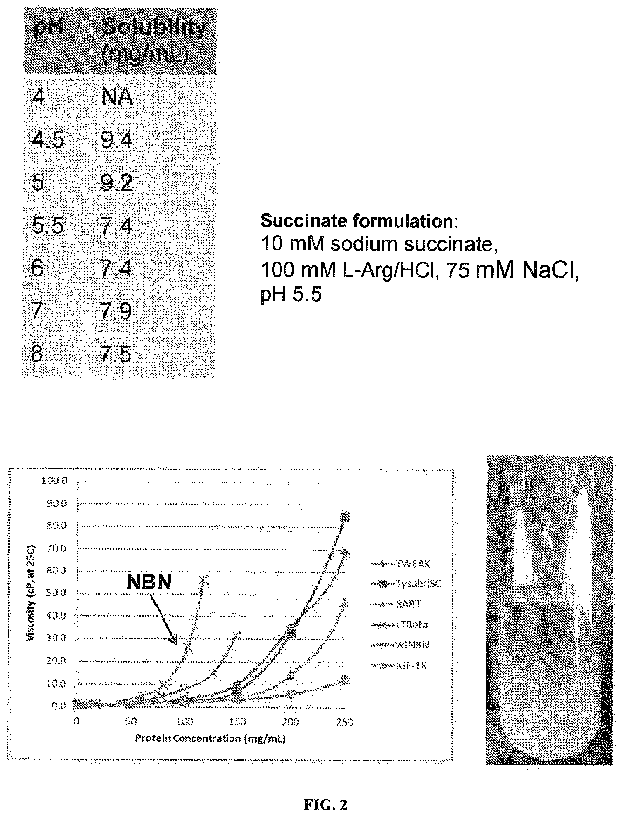

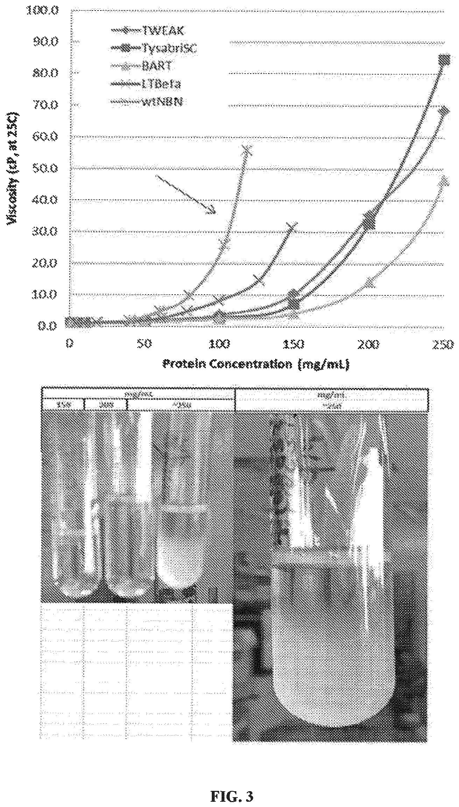

As shown in FIG. 2, the succinate formulation of Neublastin achieves a maximum solubility of 7.4 mg/mL at a pH of 5.5. Moreover, the viscosity of the Neublastin is significantly higher than other protein-based therapeutics at a given protein concentration (for example 25 cP vs. >10 cP at 100 mg/mL; FIGs. 2 and 3). The low solubility of the succinate formulation of Neublastin causes immediate phase separation of the formulation at about 200 mg/mL. At concentrations >150 mg/mL phase separation still occurs but has a longer timescale (FIG. 3).

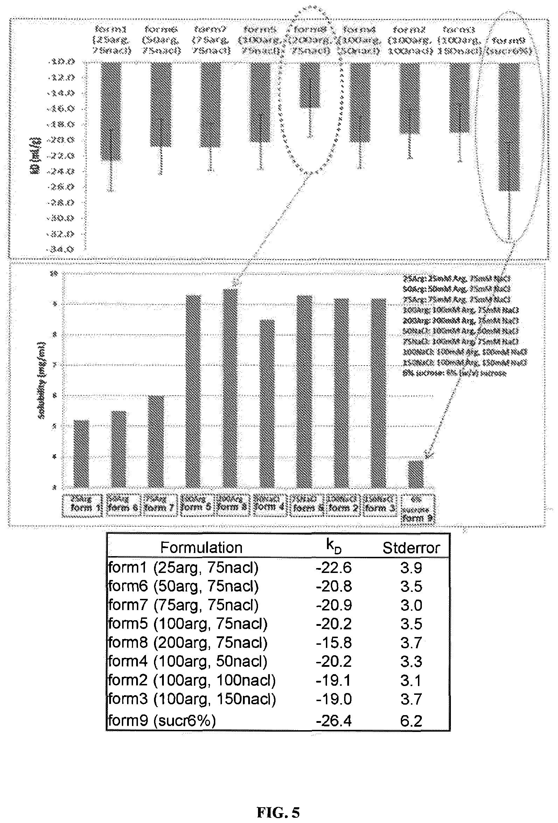

Neublastin carries a high charge at pH 5.5, as indicated by the high isoelectric point (pI) of the protein, which is generally about 11. The charge distribution of the protein may results in dipole-dipole interaction and/or other interactions that confer a high viscosity on the formulation. Based upon the information described herein, it has been discovered that the poor solubility of the succinate formulation of Neublastin correlates with unfavorable colloidal stability (k_{D}) (FIG. 5).

### Testing of new formulations of Neublastin to overcome problems associated with succinate formulation

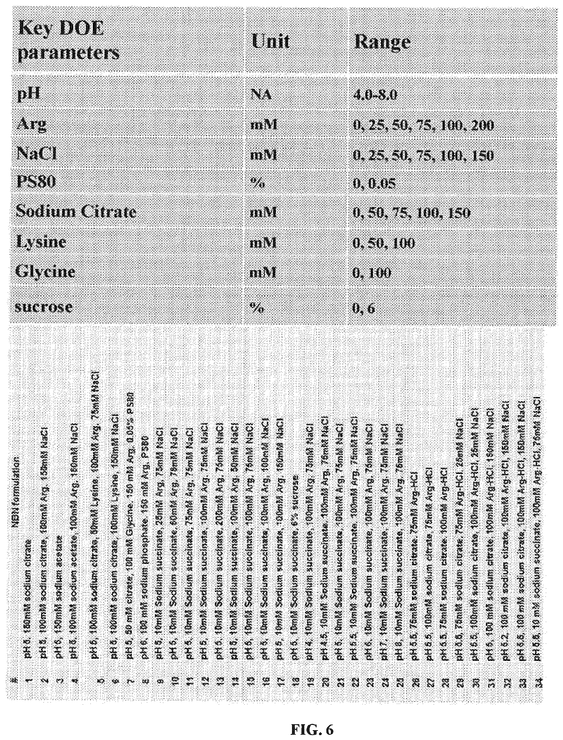

In order to address the solubility and viscosity challenges presented by the succinate formulation of Neublastin, a study was performed to assess the effects of excipients on the formulation. FIG. 6 depicts a table listing the key parameters examined in the study, as well as a list of Neublastin formulations that were tested.

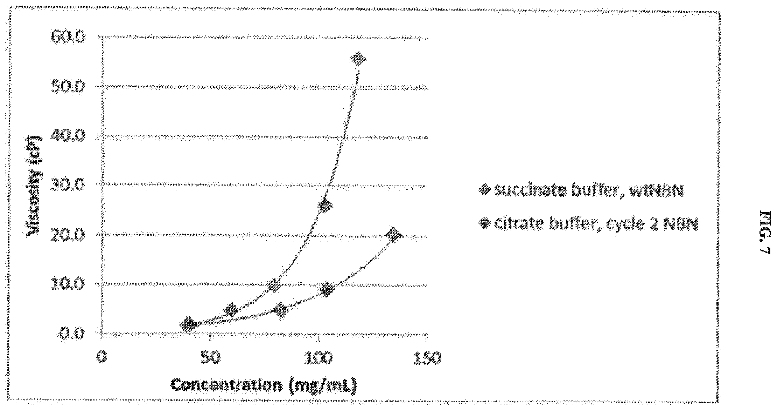

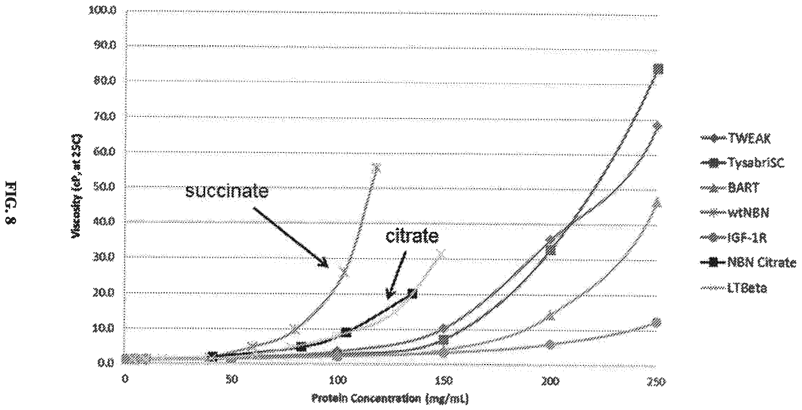

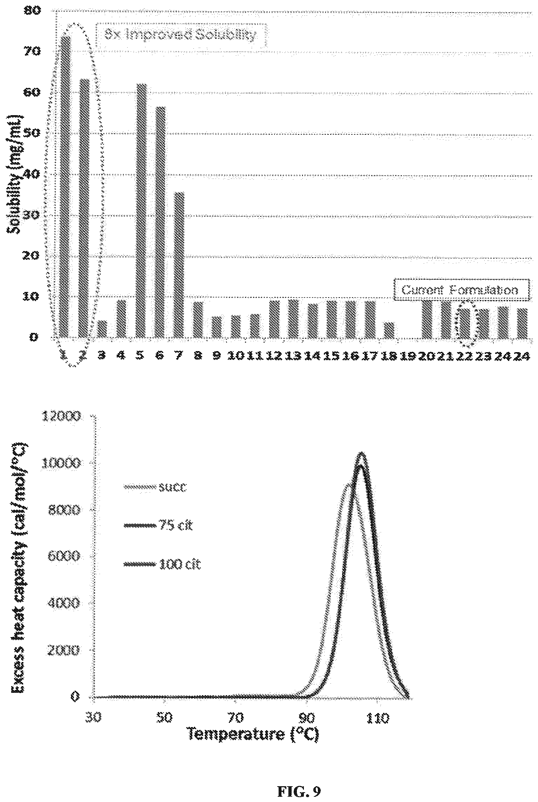

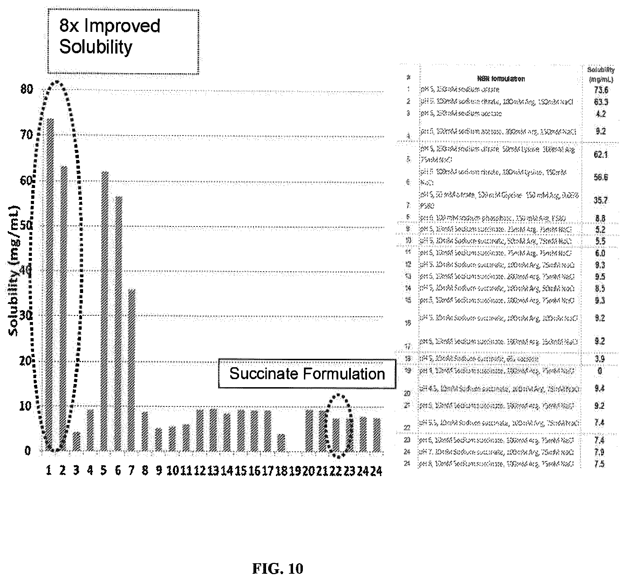

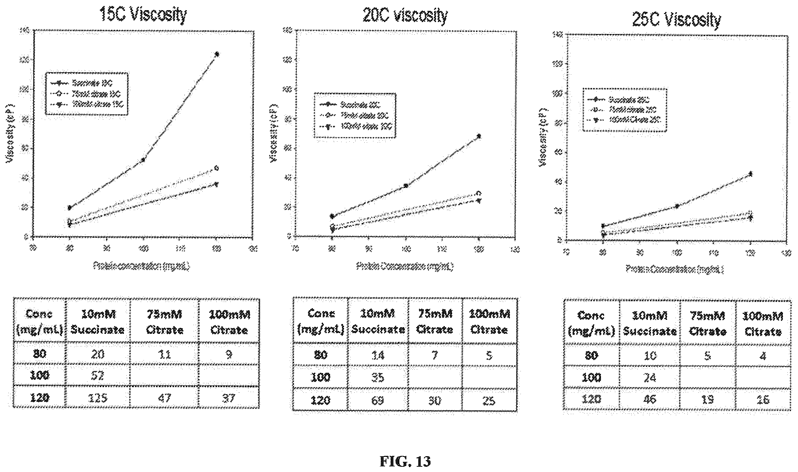

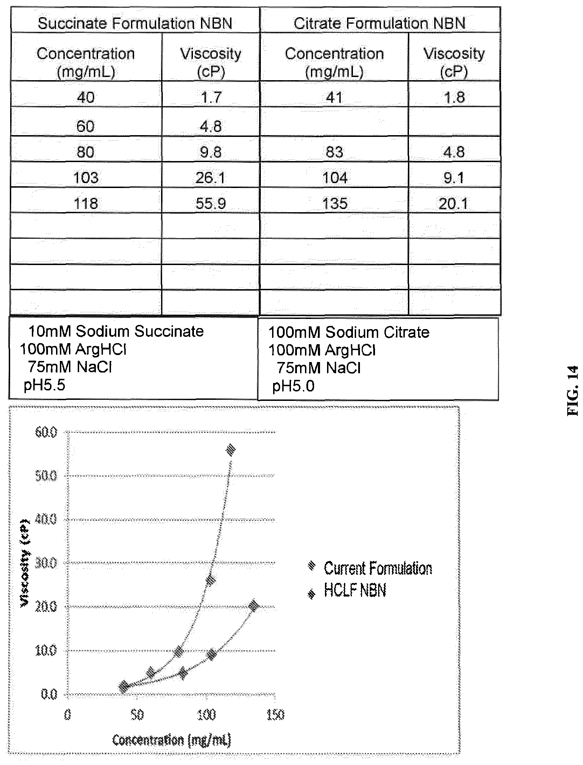

Surprisingly, the results demonstrate that replacing succinate buffer with citrate significantly improved Neublastin viscosity (FIGs. 7 and 8). The results also show that the formulation comprising Neublastin and citrate improves solubility 8-times over the succinate formulation and further increased conformational stability of Neublastin (FIGs. 9 and 10). The improvement in viscosity was observed over a range of temperatures, including after a freeze-thaw cycle (FIG. 13). Accordingly, it is believed that in some embodiments, citrate may disrupts intermolecular interaction involving (e.g., protein-protein interactions, for example, as may be caused by dipole-dipole interactions) charged Neublastin. Thus, the data indicate that the improved solubility as a result of Neublastin formulation with citrate correlates with significantly minimized viscosity (FIG. 14).

### EQUIVALENTS

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. In addition, any combination of two or more such features, systems, articles, materials, kits, and/or methods, if such features, systems, articles, materials, kits, and/or methods are not mutually inconsistent, is included within the inventive scope of the present disclosure.

All definitions, as defined and used herein, should be understood to control over dictionary definitions, definitions in documents incorporated by reference, and/or ordinary meanings of the defined terms

All references, patents and patent applications disclosed herein are incorporated by reference with respect to the subject matter for which each is cited, which in some cases may encompass the entirety of the document.

The indefinite articles "a" and "an," as used herein in the specification and in the claims, unless clearly indicated to the contrary, should be understood to mean "at least one."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, *i.e*., to mean including but not limited to. Only the transitional phrases "consisting of" and "consisting essentially of' shall be closed or semi-closed transitional phrases, respectively, as set forth in the United States Patent Office Manual of Patent Examining Procedures, Section 2111.03.

### Disclosed Items

1. A pharmaceutical formulation comprising Neublastin and citrate.
2. The pharmaceutical formulation of item 1, wherein the formulation has a viscosity suitable for subcutaneous injection.
3. The pharmaceutical formulation of item 1, wherein the formulation has a viscosity suitable for injection through a needle in a range of 29 gauge to 31 gauge in size.
4. The pharmaceutical formulation of item 1, wherein the formulation has a viscosity suitable for injection through a needle in a range of 29 gauge to 31 gauge in size while at a temperature in range of 40-60 °C.
5. The pharmaceutical formulation of item 1, wherein the formulation has a viscosity of less than 35 cP at Neublastin concentrations of up to 150 mg/mL.
6. The pharmaceutical formulation of item 1, wherein the formulation has a viscosity of up to about 20 cP at Neublastin concentrations of up to 135 mg/mL.
7. The pharmaceutical formulation of item 1, wherein the Neublastin is at a concentration in the formulation of up to 150 mg/mL.
8. The pharmaceutical formulation of item 1, wherein the Neublastin is at a concentration in the formulation in a range of above 40 mg/mL to 150 mg/mL.
9. The pharmaceutical formulation of item 1, wherein the citrate comprises citrate at a concentration of 50 mM to 150 mM
10. The pharmaceutical formulation of item 1, wherein the citrate comprises citrate at a concentration of 75 mM to 100 mM
11. A method of manufacturing Neublastin, the method comprising combining Neublastin with citrate.
12. The method of item 11, wherein the step of combining Neublastin with the citrate results in a Neublastin concentration of up to 150 mg/mL in the combination.
13. The method of item 11, wherein the step of combining Neublastin with the citrate results in a Neublastin concentration of up to 135 mg/mL in the combination.
14. The method of item 11, wherein the step of combining Neublastin with the citrate results in a Neublastin concentration of up to 100 mg/mL in the combination.
15. The method of item 11, wherein the step of combining Neublastin with the citrate results in a Neublastin concentration of above 40 mg/mL to 150 mg/mL in the combination.
16. The method of item 11, wherein the step of combining Neublastin with the citrate results in a citrate concentration of 50 mM to 150 mM in the combination.
17. The method of item 11, wherein the step of combining Neublastin with the citrate results in a citrate concentration of 75 mM to 100 mM in the combination.
18. The method of item 11, wherein the step of combining Neublastin with the citrate results in the combination having a viscosity of up to 35 cP.
19. The method of item 11, wherein the step of combining Neublastin with the citrate results in the combination having a viscosity of up to 20 cP.
20. The method of item 11, wherein the step of combining Neublastin with the citrate results in the combination having a viscosity of up to 35 cP at room temperature.
21. The method of item 11 furthering comprising filtering the combination at a temperature in a range of 30°C to 50°C.
22. The method of item 21, wherein filtering comprising performing ultrafiltration.
23. The method of item 21, wherein filtering comprising performing diafiltration.
24. A method of treating neuropathic pain in a subject, the method comprising subcutaneously administering a pharmaceutical formulation comprising a therapeutically effective amount of Neublastin and citrate to the subject.
25. The method of item 24, wherein the pharmaceutical formulation is administered through a needle of 29 gauge to 31 gauge in size.
26. The method of item 24, wherein the formulation is administered through a needle of 29 gauge to 31 gauge in size at a temperature in range of 40°C to 60 °C.
27. The method of item 24, wherein the formulation has a viscosity of less than 35 cP at Neublastin concentrations up to 150 mg/mL.
28. The method of item 24, wherein the formulation has a viscosity of up to about 20 cP at Neublastin concentrations up to 135 mg/mL.
29. The method of item 24, wherein the Neublastin is at a concentration in the formulation of up to 150 mg/mL.
30. The method of item 24, wherein the Neublastin is at a concentration in the formulation in a range of above 40 mg/mL to 150 mg/mL.
31. The method of item 24, wherein the formulation comprise citrate at a concentration of 50 mM to 150 mM
32. The method of item 24, wherein the formulation comprise citrate at a concentration of 75 mM to 100 mM
33. A method of administering Neublastin to a subject, the method comprising subcutaneously administering a pharmaceutical formulation comprising Neublastin and citrate; and
   controlling the temperature of the formulation such that the formulation is at a temperature in a range of 30 °C to 60°C during the subcutaneous administration.
34. The method of item 33, wherein the temperature is controlled such that it is in a range of 40°C to 50°C during the subcutaneous administration.
35. The method of item 33, wherein the formulation is subcutaneously administered by injecting the formulation through a needle.
36. The method of item 35, wherein the formulation is subcutaneously administered by injecting the formulation through a needle of a syringe by manually displacing a plunger of the syringe.
37. The method of item 35. wherein the formulation is subcutaneously administered by injecting the formulation through a needle that is fluidically connected to a pump configured for dispensing the formulation.
38. The method of item 33, wherein the formulation is self-administered by the subject.
39. The method of item 33, wherein the formulation is administered by a health care provider.
40. The method of item 33, wherein the formulation is administered for purposes of treating neuropathic pain.
41. A pharmaceutical formulation comprising Neublastin and a citrate salt in a lyophilized form.
42. A method comprising
   determining the extent to which the relative aqueous insolubility of a polypeptide at particular pH is associated with dipole-dipole interactions of the polypeptide, wherein the particular pH is below the isoelectric point of the polypeptide; and
   if the relative insolubility is significantly associated with dipole-dipole interactions, combining the polypeptide with citrate to enhance the solubility of the polypeptide.
43. The method of item 41, wherein the dipole-dipole interactions are intramolecular interactions.
44. The method of item 41, wherein the dipole-dipole interactions are intermolecular interactions.

## Claims

1. A pharmaceutical formulation comprising Neublastin and sodium succinate, wherein Neublastin is at a concentration in a range of 40 mg/mL to 80 mg/mL.

2. The pharmaceutical formulation of claim 1, wherein the formulation comprises L-arginine/HCl at a concentration in the formulation of 100mM.

3. The pharmaceutical formulation of claim 1 or 2, wherein the sodium succinate is at a concentration of 10mM.

4. The pharmaceutical formulation of any of claims 1-3, wherein the formulation is at a pH in the range of 4,5 - 8, preferably 5,5 - 8.

5. The pharmaceutical formulation of any of claims 1-4, wherein the formulation further comprises NaCl at a concentration of 75mM.

6. A method of manufacturing a Neublastin formulation of claim 1, the method comprising combining Neublastin with sodium succinate, wherein the Neublastin is at a concentration in a range of 40 mg/mL to 80 mg/mL.

7. A pharmaceutical formulation for use in treating neuropathic pain in a subject, the pharmaceutical formulation comprising a therapeutically effective amount of Neublastin and sodium citrate, wherein the Neublastin is at a concentration in a range of 40 mg/mL to 80 mg/mL.
